# EUROPÄISCHE PATENTANMELDUNG

(11) **EP 0 808 905 A2**
(43) Veröffentlichungstag der Anmeldung: **26.11.1997**
(21) Anmeldenummer: 97107146.9
(22) Anmeldetag: 30.04.1997
(51) Int. Cl.: C12N 15/86, A61K 48/00

(54) **Neuer adenoviraler Vektor für den Transfer humaner Gene in vivo**

(30) Priorität: 22.05.1996 DE 19620687
(71) Anmelder: Centeon Pharma GmbH, 35002 Marburg (DE)
(72) Erfinder: Poller, Wolfgang Christian, Dr., 97078 Würzburg (DE)

(57) **Zusammenfassung**

Die Erfindung betrifft einen neuen adenoviralen Vektor für den Transfer humaner Gene in vivo. Anwendungsgebiete der Erfindung sind die Medizin und die pharmazeutische Industrie.

## Beschreibung

Die Erfindung betrifft einen neuen adenoviralen Vektor für den Transfer humaner Gene in vivo. Anwendungsgebiete der Erfindung sind die Medizin und die pharmazeutische Industrie.

Die medikamentöse Behandlung der schweren monogenen Erbkrankheiten Hämophilie A bzw. B basiert auf der Substitution der den Erkrankten fehlenden Gerinnungsfaktoren Faktor VIII bzw. IX per intravenöser Infusion, die im Normalfalle etwa 3 Mal pro Woche erfolgen muß. Im Falle der Bildung von Hemmkörpern gegen die substituierten Gerinnungsfaktoren, ebenso auch im Falle erhöhten Faktorenverbrauchs (etwa im Rahmen von operativen Eingriffen am Patienten), ist teilweise eine mehrmals tägliche Gabe der Faktoren erforderlich. Dieses Prozedere, das lebenslang beizubehalten ist, ist für den Patienten psychologisch belastend. Es wäre unter diesem Aspekt wünschenswert, wenn ein therapeutisches Verfahren zur Verfügung stünde, das deutlich größere Behandlungsintervalle (etwa in der Größenordnung von Monaten) zuließe. Mit exogen zugeführten Faktoren ist dies jedoch nicht möglich, da diese im Organismus des Empfängers nur eine kurze Halbwertzeit besitzen.

Ein weiterer Aspekt der heutigen Hämophilietherapie betrifft die Sicherheit des Verfahrens. Bei Faktorenkonzentraten aus humanem Spender-Poolplasma besteht im Prinzip die Möglichkeit, daß bisher nicht bekannte oder nicht sicher identifizierbare infektiöse Agenzien mitübertragen werden, obwohl zwischenzeitlich eingeführte Inaktivierungsverfahren sowie die einzelnen Reinigungsschritte selbst solche Agenzien inaktivieren bzw. entfernen. Die Verwendung von in eukaryotischen Zellinien hergestellten rekombinanten humanen Gerinnungsfaktoren reduziert ein etwaiges Risiko ganz erheblich, wenn auch die Existenz latenter kryptogener Viren in den Produktionszellinien nicht absolut ausgeschlossen werden kann. Eine Verlängerung des Behandlungsintervalls läßt sich jedoch auch mit den rekombinanten Faktoren nicht erreichen.

Mit der Verfügbarkeit von Verfahren für den Transfer klonierter Gene in vivo, also im intakten Organismus, hat sich die prinzipielle Möglichkeit einer sogenannten "Somatischen Gentherapie" der Hämophilien eröffnet. Falls es gelingt, 1. die humanen Gene für Faktor VIII. bzw. IX. mit hoher Effizienz in den Patienten einzubringen (vorzugsweise in die Leber als den physiologischen Syntheseort) und 2. deren Expression durch einmalige Applikation oder durch wiederholte Applikation in größeren Zeitabständen (mehrere Wochen bis Jahre) dauerhaft zu sichern, so wäre dies ein ganz wesentlicher Fortschritt in der Behandlung von Hämophilie-Patienten, wenn keine relevanten Nebenwirkungen auftreten.

Verschiedene Gentransfersysteme sind seit 1990 entwickelt worden mit dem langfristigen Ziel ihrer Anwendung für Zwecke der Somatischen Gentherapie. Eines der vielversprechendsten System sind die sogenannten replikationsdefekten adenoviralen Vektoren, die nicht nur ex vivo, sondern auch in vivo im intakten Gesamtorganismus eine sehr hohen Gentransfereffizienz gewährleisten, wie sie mit keinem anderen derzeit verfügbaren System erreicht werden können. Das adenovirale Vektorsystem in seiner bisherigen Form hat allerdings noch zwei gravierende Mängel. Zum einen ist die Expressionsdauer in vivo limitiert (in den meisten Modellsystemen auf wenige Wochen), zum anderen löst der Vektor selbst im Empfänger immunologische Reaktionen aus, die nicht nur die Vektorelimination aus dem Zielgewebe beschleunigen, sondern dort auch zu immunpathologischen Phänomenen führen. Falls die oben skizzierten Probleme des adenoviralen Systems überwunden werden könnten, so wäre dies ein wesentlicher Fortschritt auf dem Weg zu einer langfristigen Gentherapie monogener und anderer Krankheiten.

Ziel der Erfindung ist die Weiterentwicklung des adenoviralen Vektorsystems mit dem Ziel der Überwindung der genannten Schlüsselprobleme. Die Aufgabe der Erfindung besteht darin, den Transfer von Genen mittels adenoviraler Vektoren in die Leber so zu gestalten, daß eine gegenüber den bisherigen Verfahren erheblich verlängerte Transgenstabilität erreicht wird.

Die Erfindung wird gemäß den Ansprüchen 1 bis 6 realisiert, die Unteransprüche sind Vorzugsvarianten.

Kernstück der Erfindung ist die Konstruktion eines neuen replikationsdefekten adenoviralen Vektors, der als wichtiges Element die vollständige E3-Region von Wildtyp-Adenovirus (vorzugsweise Serotyp 5) enthält, ggfls. mit spezifischen Veränderungen dieser Region mit dem Ziel, die Expression von E3-Genen zu verstärken (durch Einbau starker, konstitutionell aktiver Promotor wie den des Cytomegalovirus) und die Verwendung dieses Vektors zum Gentransfer unter gleichzeitiger transienter anti-CD4 Behandlung des Empfängerorganismus. Die Anti-CD4-Behandlung wird vorzugsweise mit geeigneten monoklonalen Antikörpern gegen CD4-Antigen durchgeführt, die zeitlich mit der Applikation des adenoviralen Vektors für den Gentransfer überlappt. Wesentliches Element der Erfindung ist die Kombination des E3-positiven Vektors mit der anti-CD4-Strategie zur Verbesserung des hepatischen Gentransfers.

Geeignete monoklonale Anti-CD4-Antikörper sind beispielsweise solche, die die Signaltransduktion vom CD4-Rezeptor blockieren oder den Zielorganismus von CD4-positiven Lymphozyten depletieren. Besonders bevorzugt sind jeweils entsprechende humanisierte monoklonale Antikörper.

Die Weiterentwicklung des adenoviralen Vektorsystems gemäß der Erfindung ermöglicht eine erhebliche Verbesserung der Therapie verschiedener Erkrankungen. Die monogenen Erbkrankheiten Hämophilie A bzw. B sollten mit Hilfe solcher Vektoren (E3-positiver, E3-verstärkter) in Kombination mit transienter Anti-CD4-Behandlung zur Toleranzinduktion besser behandelt werden können.

Die Erfindung wird nachfolgend durch Ausführungsbeispiele näher erläutert.

### Beispiel 1. Aufbau des Vektors und Einbau des Gens

### A. Einbau humaner F IX cDNA in ein Expressionsplasmid.

F IX cDNA wurde mittels Reverser Transcriptase gekoppelter Polymerase-Kettenreaktion (RT-PCR) aus Gesamt RNA von humaner Leber hergestellt, wie im folgenden beschrieben. Die mit RNeasy® (Quiagen Inc.) isolierte poly A⁺ mRNA wurde mittels Oligotex® (Quiagen Inc.) aus der Gesamt RNA gereinigt und für die Synthese der codierenden cDNA mittels AMV RT (Boehringer Mannheim ) verwendet. Darauf schloß sich die RT-PCR an unter Verwendung von thermostabiler AmpliTaq® DNA Polymerase (Applied Biosystems Inc.). Die sich ergebende DNA wurde zunächst in ein PCR-Script® Plasmid (Stratagene Inc.) kloniert und anschließend sequenziert. Die durch Sequenzierung verifizierte F IX-cDNA wurde aus dem o. g. Klonierungsvektor herausgeschnitten und in das für eukaryotische Zellen geeignete Expressionsplasmid pZS2 insertiert; Plasmid pZS2 enthält als hier wesentliche Bestandteile eine Adenovirus-Sequenz von der 5'-ITR-Sequenz bis zur Nucleotidposition 445, gefolgt von dem CMV Promotor, einer Polylinkersequenz, Terminationssignalen des bovinen Wachstumshormongens sowie einer einzigen XbaI Restriktionssequenzstelle, die dazu benutzt wird, das linearisierte F IX / pZS2 Plasmid in den Adenovirusvector RR5 (s. u.) einzuklonieren. Das F IX-Strukturgen ist dabei so einligiert, daß es unter Kontrolle des starken CMV Promotors / Enhancers steht.

### B Aufbau der E3⁺-Adenoviruskomponente

Quelle für das hier geeignete Adenovirusgenom war das RR5-Virus, der eine Deletion in der E1-Region enthält (von Nukleotid-Position 445 bis 3333). Durch diese Deletion wird das Virus defizient bezüglich Replikation. RR5 hat jedoch die gesamte E3-Region des Adenovirus Typ 5 (Ad 5) beibehalten.
RR5 wurde in menschlichen embryonalen Nierenzellen Stamm 293 vermehrt und nach Isolierung über CsCl-Dichtegradientenzentrifugation mit anschließender Entsalzung über Sephadex® (Pharmacia AB) G-25 Säulenchromatographie erhalten. Virale DNA wurde aus der vorgenannten Viruspräparation isoliert und mit Xba I Restriktionsendonuklease geschnitten, was das Genom in ein großes 3'-terminales und ein kleineres 445 kb 5' terminales zerlegt.

Das recombinante linearisierte F IX/pZS2 Plasmid wird nun mit dem großen XbaI Fragment aus RR5 ligiert und in den o. g. Nierenzellen vermehrt, nachdem diese mit dem Ligationsprodukt per Calciumphosphatmethode transfeziert waren. Ausgehend von Einzelplaques mittels der bekannten Standard-Plaques-Reinigungsmethode und Kontrolle über F IX-spezifische PCR-Analyse wurde schließlich der gesuchte Vektor Ad 5E3⁺ΔE1 F IX erhalten.

### Beispiel 2. Vektorexpression in primären humanen vasculären Endothelial-Zellen (HUVEC)

Zur Kontrolle wurden jeweils konfluente primäre humane Endothel-Zellkulturen (HUVEC), die in Kultur bis zu 4 Wochen stabil waren, bei einer Multiplizität der Infektion (MOI) von 10 mit einem Adenovirus-Luciferasevektor oder dem das Faktor IX Gen tragende Vektor Ad5E3⁺ F IX infiziert. Sowohl F IX:Ag bzw. F IX:c Aktivität als auch Luciferase-Aktivität waren mit jeweils vergleichbaren Expressionskinetiken nachweisbar über 4 Wochen, ohne daß an den Endothelzell-Monolayers hierdurch pathologische Veränderungen induziert wurden.

### Beispiel 3. Langzeitexpression des E3⁺ F IX-Vektors in der Maus

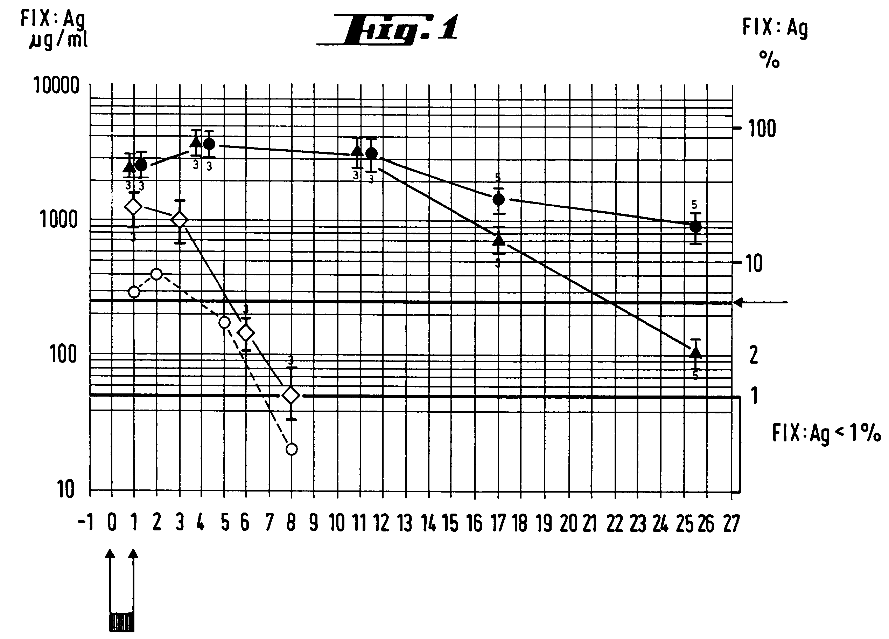

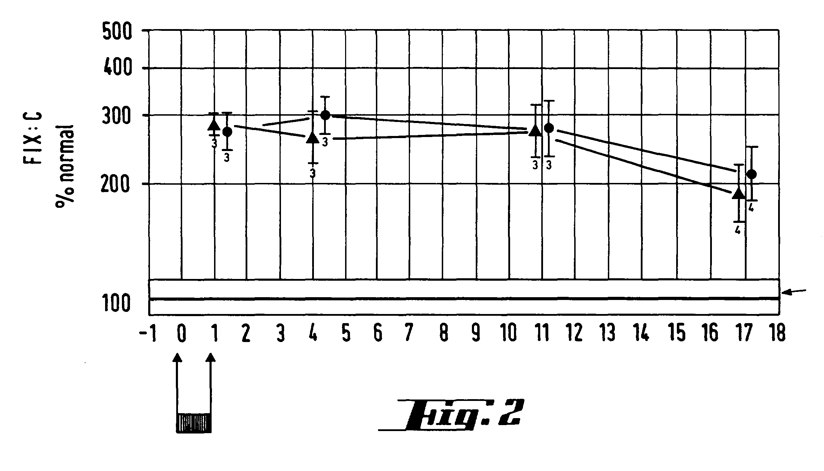

Der die DNA für humanen Factor IX enthaltende Vektor Ad5E3⁺ F IX wurde in Mäusen mit einer Dosis von 2 x l0¹⁰ PFU pro Tier intravenös injiziert. Insgesamt 20 Mäuse wurden so behandelt, wobei 10 davon zusätzlich transiente Anti-CD4 Behandlung erhielten. Adenovirale Vektoren erkennen als Zielorgan die Leber; die Expressionsaktivität des F IX Transgens in der Leber wurde im Plasma der mit Vektor injizierten Mäuse mittels Bestimmung des rekombinanten humanen F IX:Ag in einem speziesspezifischen ELISA gemessen. Die Ergebnisse, in Prozent des Werts in Normalhumanplasma (100 % Wert bei 5000 µg/ml) sind in Fig. 1 dargestellt. Die Faktor IX:Ag-Konzentrationen, die zu Beginn bei den unbehandelten Mäusen < 1 % (< 50 µg/ml) betrugen, stiegen bis auf 120 % (6000 µg/ml) in einzelnen Tieren an. Alle behandelten Mäuse besaßen Konzentrationen an F IX:Ag in der Nähe der physiologischen Werte während der ersten drei Monate der Studie (Fig. 1).
Der rekombinante F IX war auch funktionell aktiv, was durch einen Test auf F IX Gesamtaktivität (F IX:C) im Mausplasma mit Hilfe eines chromogenen Substrats gezeigt wurde (Fig. 2). Der Basiswert unbehandelter Mäuse betrug 102 ± 8 %. Die F IX:C-Werte nach dem F IX-Gen-Transfer sind als Prozentwert der Aktivität von normalem humanem Pool-Plasma (= 100 %) angegeben. Nach Behandlung mit Vektor Ad5E3⁺ F IX stiegen die Werte von F IX:C in Mausplasma schnell auf Werte bis 320 % in einzelnen Tieren. Die zeitlichen Verlaufskurven sind dabei ähnlich wie die bei rekombinantem F IX:Ag.

Das humane F IX:Ag erreichte Werte um 60 % (3000 µg/ml) schon nach einer Woche post Injektion. Sowohl in den immunokompetenten als auch den anti-CD4 behandelten Tieren lagen die humanen F IX:Ag-Werte in ähnlicher Höhe ziemlich gleichmäßig in der Nähe der physiologischen Werte bis zu 10 Wochen nach dem Gen-Transfer und blieben deutlich über dem therapeutischen Schwellenwert von 5 % (250 mg/ml) während insgesamt 4 Monaten.

Mit dem in einer früheren Arbeit (Smith et al. "Adenovirus mediated expression of therapeutic plasma levels of human factor IX in mice", Nature Genet 1993, 5: 397-402) eingesetzten E3-deletierten Vektor (Av1H9B) der ebenfalls das humane F IX-Gen enthält und in denselben Mausinzuchtstamm C57B1/6 wie in den vorangegangenen Experimenten transfeziert wurde, sind die F IX-Expressionswerte ebenfalls in Fig. 1 dargestellt (gestrichelte Linie, offene Kreise). Eine ganz ähnliche Kurzzeitexpression wurde mit dem Vektor Ad5ΔE3 F IX erhalten, dem vollständig E3-deletierten Gegenstück des zuerst geschilderten Vektors mit E3-Region. Bereits nach etwa 6 Wochen war die Expression unter den therapeutischen Schwellenwert gefallen (Fig. 1, durchgezogene Linie mit Rhomben).
Dieser direkte Vergleich eines E3-positiven vs. E3-negativen F IX-Vektors demonstriert den Transgen-stabilisierenden Effekt von E3.

Ein weiteres wesentliches, neues Ergebnis dieser in vivo-Langzeitversuche war die Beobachtung, daß der Vektor Ad5E3⁺ F IX in transient anti-CD4-behandelten Empfängern eine hochsignifikant verbesserte Langzeitstabilität zeigte. Die Faktor IX-Plasmaspiegel in der anti-CD4-Gruppe lagen sogar 1/12 Jahr nach dem Gentransfer noch vierfach über der therapeutischen Schwelle von 5 %, während zu diesem Zeitpunkt die Kontrollgruppe bereits auf subtherapeutische Levels abgefallen war.

### Legende zu Fig. 1 und Fig. 2:

Ausgefüllte Dreiecke stellen die Verlaufskurve bei mit F IX-Gen tragenden Vektor behandelten immunkompetenten Mäusen dar, ausgefüllte Kreise die Verlaufskurve bei zusätzlicher Behandlung mit anti-CD4 Antikörper (YTS 191). Die offenen Dreiecke bzw. Kreise stellen die Verlaufskurve in Mäusen dar, die jeweils mit E3-deletierten Vektoren erhalten werden.

Der Pfeil rechts und die durchgezogene Linie dazu markiert den therapeutisch wirksamen F IX:Ag Wert.

Die durchgezogene Basislinie stellt den F IX:Ag Wert in unbehandelten Kontrollmäusen dar.

Die Zahlen der Abszisse geben die Wochen nach Vektorinjektion an und die Pfeile bei 0 und 1 den Zeitpunkt der Anti-CD4-Behandlung.

## Patentansprüche

1. Adenoviraler Vektor für den Gentransfer, dadurch gekennzeichnet, daß er die partielle oder vollständige E3-Region des Wildtyp-Adenovirus Typ 5 sowie das zu transferierende Gen mit therapeutischem Potential enthält.

2. Adenoviraler Vektor Ad5E3⁺ΔE1nach Anspruch 1.

3. Adenoviraler Vektor nach Anspruch 1 oder 2, dadurch gekennzeichnet, daß das Gen für den humanen Gerinnungsfaktor IX integriert ist.

4. Adenoviraler Vektor nach Anspruch 1 oder 2, dadurch gekennzeichnet, daß das Gen für humanes Endothelin-1 integriert ist.

5. Adenoviraler Vektor nach Anspruch 1 oder 2, dadurch gekennzeichnet, daß das Gen für α1-Antitrypsin integriert ist.

6. Adenoviraler Vektor nach Anspruch 1 oder 2, dadurch gekennzeichnet, daß das Gen für den humanen Gerinnungsfaktor VIII oder ein funktioneller Teil davon integriert ist.

7. Kombinationspräparat, enthaltend einen adenoviralen Vektor nach Anspruch 1 - 6 und ein Mittel zur Anti-CD4-Behandlung.

8. Kombinationspräparat nach Anspruch 7, dadurch gekennzeichnet, daß das Mittel zur Anti-CD4-Behandlung eine Rezeptorblockade oder eine T-Zell-Depletion bewirkt.
